# EUROPEAN PATENT APPLICATION

(11) **EP 2 258 442 A1**
(43) Date of publication of application: **08.12.2010**
(21) Application number: 09014480.9
(22) Date of filing: 20.11.2009
(51) Int. Cl.: A61N 1/08, A61N 1/375, A61N 1/39, H01L 21/00

(54) **Dielectric fluid filled active implantable medical devices**

(30) Priority: 03.06.2009 US 477422
(71) Applicant: Greatbatch Ltd., Clarence, NY 14031 (US)
(72) Inventor: Stevenson, Robert A., Canyon Country, California 91387 (US)
(74) Representative: Richter, Werdermann, Gerbaulet & Hofmann

(57) **Abstract**

Active implantable medical devices (AIMDs) are backfilled with a dielectric fluid to increase the volts per mil dielectric breakdown strength between internal circuit elements. In a method for backfilling the AIMD with dielectric fluid, substantially all air and moisture is evacuated from the AIMD housing prior to backfilling the AIMD housing with a dielectric fluid having a dielectric breakdown strength greater than air, nitrogen or helium. The AIMD is constructed to accommodate volumetric expansion or contraction of the dielectric fluid due to changes of pressure or temperature of the dielectric fluid to maintain integrity of the AIMD.

## Description

### BACKGROUND OF THE INVENTION

This invention relates generally to active implantable medical devices (AIMDs). More particularly, the present invention relates to fluid-filled active implantable medical devices which increase the volts per mil (volts-per-thousandths of an inch) breakdown strength of circuit elements housed within such devices.

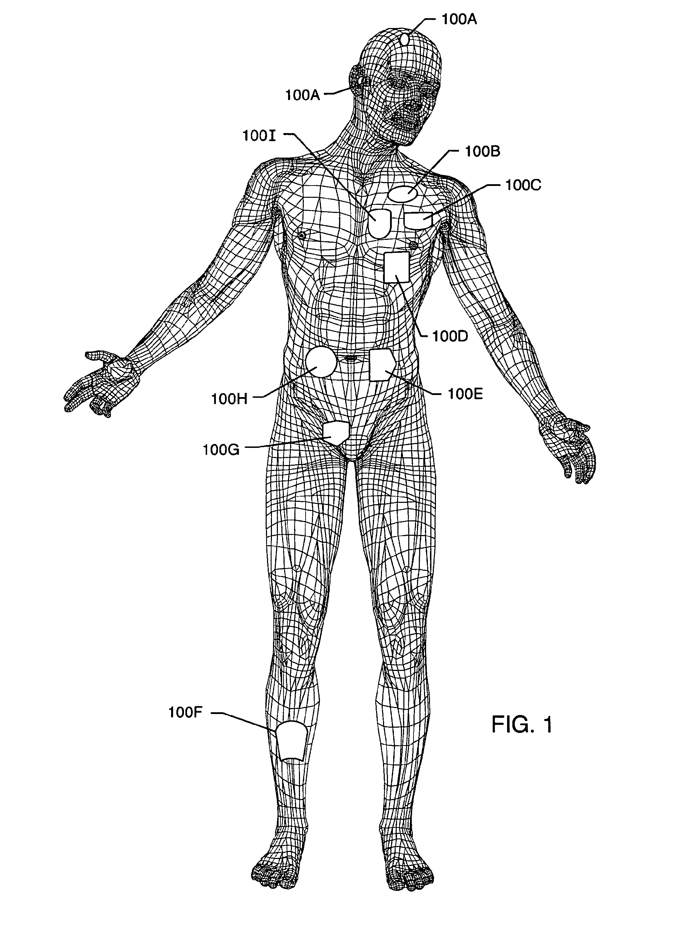

Active implantable medical devices (AIMDs) generally consist of an enclosure, such as titanium, which houses electronic circuits. Typically, there is a hermetic seal associated with lead wires which are implanted into body tissue. FIG. 1 is a wire formed diagram of a generic human body showing a number of implanted medical devices. 100A represents a family of hearing devices which can include the group of cochlear implants, piezoelectric sound bridge transducers and the like. 100B represents a variety of neurostimulators and brain stimulators. Neurostimulators are used to stimulate the Vagus nerve, for example, to treat epilepsy, obesity and depression. Brain stimulators are pacemaker-like devices and include electrodes implanted deep into the brain for sensing the onset of the seizure and also providing electrical stimulation to brain tissue to prevent the seizure from actually occurring. 100C shows a cardiac pacemaker which is well-known in the art. 100D includes the family of left ventricular assist devices (LVAD's), and artificial hearts, including the recently introduced artificial heart known as the Abiocor. 100E includes an entire family of drug pumps which can be used for dispensing of insulin, chemotherapy drugs, pain medications and the like. 100F includes a variety of bone growth stimulators for rapid healing of fractures. 100G includes urinary incontinence devices. 100H includes the family of pain relief spinal cord stimulators and antitremor stimulators. 100H also includes an entire family of other types of neurostimulators used to block pain. 100I includes a family of implantable cardioverter defibrillators (ICD) devices and also includes the family of congestive heart failure devices (CHF). This is also known in the art as cardio resynchronization therapy devices, otherwise known as CRT devices.

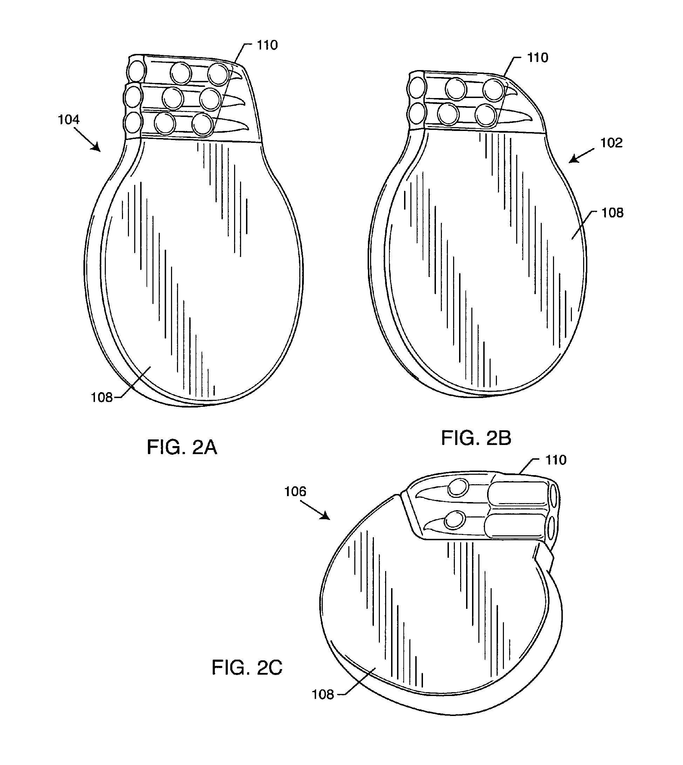

FIG. 2A-2B illustrate prior art implantable cardioverter defibrillators 102, and 104. The smaller defibrillator 102 does not have biventricular capabilities. The housing 108 of the implantable cardioverter defibrillators (ICD) 102 and 104 is typically of a thin-wall titanium construction. The larger ICD 104 has additional ports in the header block assembly 110 allowing for leads to be placed in the right ventricle, the right atrium and also leads to be placed through the coronary sinus in the great cardiac vein to the outside of the left ventricle. FIG. 2C is very similar to FIG. 2B except that this is a much smaller implantable cardiac pacemaker 106. The cardiac pacemaker 106 also typically has a thin-wall titanium housing 108 as shown. In this case, the header block 110 is suitable for plugin connections of cardiac leads.

These AIMD housings are normally backfilled with dry nitrogen and then sealed. Air is generally not used because of its propensity to contain moisture. An advantage of prior art backfilling with dry nitrogen is that the nitrogen is very light in weight and adequately displaces air within the enclosed volume of the AIMD. The process of putting in the nitrogen generally involves heating the device and applying a vacuum which draws out all the air molecules and associated moisture. The device is then flooded in dry nitrogen and the vacuum is cracked and replaced with pressure. This has the effect of driving the nitrogen into all the spaces inside of the AIMD.

However, a drawback of nitrogen or other such backfill gases is that they have very poor dielectric breakdown properties. That is, they have a relatively low breakdown strength that is closely associated with that of air. This is particularly disadvantageous in high voltage devices such as implantable cardioverter defibrillators. For example, in a typical nitrogen gas backfilled ICD application, circuit traces are kept at a minimum distance of 25 millimeters apart from each other. This is in order to avoid the potential for arcing, ionization of the gas (such as nitrogen) which could lead to catastrophic failure of the AIMD.

In physics, the term dielectric strength has the following meanings:
(1) Of an insulating material, the maximum electric field strength that it can withstand intrinsically without breaking down, i.e., without experiencing failure of its insulating properties.
(2) For a given configuration of dielectric material and electrodes, the minimum electric field that produces breakdown.
(3) The maximum electric stress the dielectric material can withstand without breakdown.

The theoretical dielectric strength of a material is an intrinsic property of the bulk material and is dependent on the configuration of the material or the electrodes with which the field is applied. At breakdown, the electric field frees bound electrons. If the applied electric field is sufficiently high, free electrons may become accelerated to velocities that can liberate additional electrons during collisions with neutral atoms or molecules in a process called avalanche breakdown. Breakdown occurs quite abruptly (typically in nanoseconds), resulting in the formation of an electrically conductive path and a disruptive discharge through the material. For solid materials, a breakdown event severely degrades, or even destroys, its insulating capability.

Factors affecting dielectric strength:
(1)It increases with the increase in thickness of the specimen. (Directly proportional)
(2)It decreases with the increase in operating temperature. (Inversely proportionable)
(3) It decreases with the increase in frequency. (Inversely proportionable)
(4) It decreases with the increase in humidity. (Inversely proportionable)

The field strength at which breakdown occurs in a given case is dependent on the respective geometries of the dielectric (insulator) and the electrodes with which the electric field is applied, as well as the rate of increase at which the electric field is applied. Because dielectric materials usually contain minute defects, the practical dielectric strength will be a fraction of the intrinsic dielectric strength seen for ideal, defect free, material. Dielectric films tend to exhibit greater dielectric strength than thicker samples of the same material. For instance, dielectric strength of silicon dioxide films of a few hundred nm to a few µm thick is approximately 0.1 MV/m. Multiple layers of thin dielectric films are used where maximum practical dielectric strength is required, such as high voltage capacitors and pulse transformers.

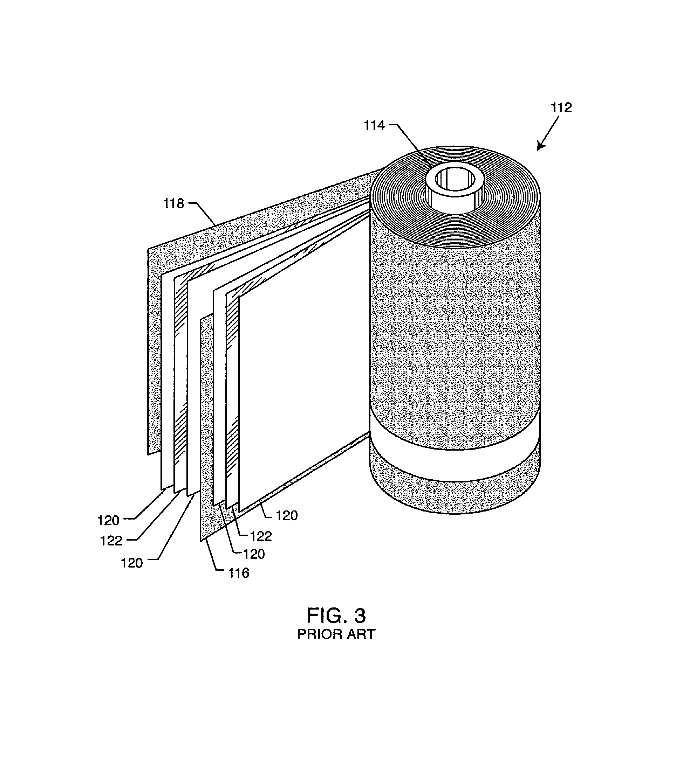

It is commonly known in the prior art to backfill transformers and large power line capacitors with a dielectric liquid such as chlorinated hydrocarbons, aerochlor, mineral oils, silicone oils and the like. There are a number of dielectric fluids that are commonly used. In this regard, FIG. 3 illustrates a prior art layer wound feedthrough capacitor 112. The layers are wound around a center tube 114 which is typically of paper, plastic or the like. The sandwich construction consists of capacitor electrodes typically of extruded aluminum foil or aluminum foil which has been sprayed and metalized. The bottom or ground electrode, in this case, is shown as 116. The active electrode 118 extends upwardly and makes contact at the top of the capacitor as shown. A number of materials can be interleaved between the electrode plates 116 and 118. Plastic film (Mylar) 120 is typically used in the prior art, as well as interleaves of paper 122. There are other materials in addition to paper that can be used. What is important here is that a porous material suitable for impregnation be used. This type of capacitor, when it does involve a porous dielectric, such as paper 122, is designed to be liquid impregnated. The reason for this is that air in the paper pores has a relatively low dielectric breakdown strength and also a relatively low permittivity. This means that until the capacitor 112 is properly impregnated with the dielectric fluid, it will not be very efficient for storing charge or for standing off high voltage.

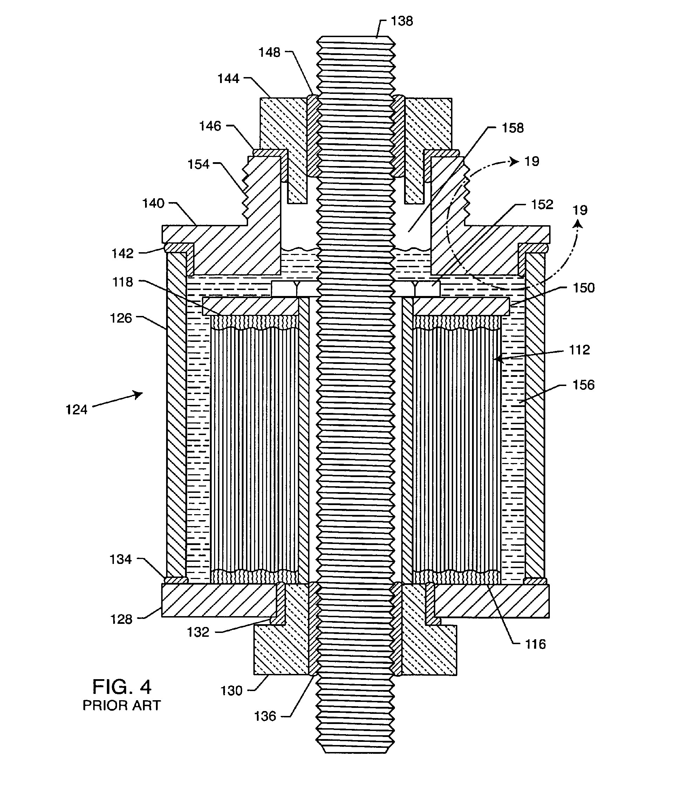

FIG. 4 shows the prior art layer wound feedthrough capacitor 112 of FIG. 3 installed in a metal housing 124. The metal housing 124 is typically a metal-plated steel tube 126. It has an end cap 128 which is also metal. A ceramic seal 130 is soldered or brazed 132 to the end cap 128. A hermetic seal is further formed by virtue of the hermetic seal joints 134 and 136. In this case there is a threaded rod 138 that runs entirely through the center of the layer wound capacitor 112. A threaded end bushing 140 is attached to the other end of housing 124. This is attached by soldering or brazing at joint 142. A second alumina ceramic insulator 144 forms a mechanical and hermetic seal at points 146 and 148. This type of construction makes it into a very highly efficient feedthrough-type capacitor useful for EMI filtering of a broad range of frequencies.

In typical prior art construction, the entire mechanical assembly would be assembled together, including the installation of the layer wound capacitor 112, and then a conductive washer 150 is placed down and a nut 152 is torqued so that the washer 150 makes intimate electrical contact with the extruded aluminum electrode 118. On the opposite end, the ground electrode 116 is mechanically and electrically compressed and coupled to the plated end plate 128. This completes the electrical circuit. Electrical noise that would be traveling down threaded rod 138 would be efficiently decoupled by the feedthrough capacitor assembly 112 to the ground plane (not shown). The connection to a shield or ground plane is typically made by inserting the threaded portion 154 of the end bushing 140 through the hole in a bulk head and attaching it securely with a nut. In this way, electromagnetic signals are decoupled from threaded rod 138 to the ground plane.

This assembly normally has an open joint left for impregnation of various types of dielectric fluid 156. All of the joints as shown in FIG. 4 would typically be preconstructed with the exception of one, which could be left entirely out or, alternatively, a pin hole through it could be left for suitable impregnation. During the impregnation process, the entire assembly is typically first lowered into a high temperature vacuummaking chamber, such as made by Redpoint Industries. At elevated temperature and over a period of time, a hard vacuum is pulled such that the paper 122 that is sandwiched in between the capacitor electrodes is free of all the trapped air. Then the chamber is flooded with a dielectric fluid 156 over the top of all of the feedthrough capacitor assembly. Then, in an optimal impregnation process, positive pressure is applied over the top of the dielectric fluid. This is best done with dry nitrogen so that no moisture is introduced into the system. Typical pressures can range from 40 to 100 psi or even higher. The capacitor assembly is allowed to sit in this condition usually for several hours. This is a very efficient impregnation process in that inside the paper of the layer wound capacitor, there is literally a vacuum which tends to pull the fluid inside. The positive pressure on top of the fluid assists this process. So over time, the interior of the capacitor is completely filled with the dielectric fluid 156.

In the past, dielectric fluids included chlorinated hydrocarbons, such as Aerochlor. There were other types of chlorinated askarels that were also used. Most of these have been banned today due to environmental concerns. Popular impregnates today are silicone oils, mineral oils and even high dielectric breakdown gasses, such as sulfur hexaflouride.

There was a problem when constructing the types of devices of FIG. 4 for the Minute Man Missile System. In particular, when the unit was completely filled with dielectric fluid 156, there was no entrapped air cavity 158 left behind. In other words, when the units were taken out of the impregnation chamber (still warm) and last remaining hermetic seal joint was formed, the unit was 100% liquid filled. During temperature excursions (increases), the liquid 156 would expand and tend to break either one of the hermetic seals 130 or 144 or possibly fracture a solder joint. This caused leakage of the dielectric fluid 156 and led to reliability problems. Accordingly, it was discovered that the units should be slightly drained to deliberately leave an air cavity 158, which allowed a space for the liquid 156 to expand and contract so that it could not damage the important hermetic seals. However, the presence of the air space 158 led to an undesirable air bubble. As the assembly was tilted, this could cause an air bubble to occur between points of high voltage. For example, if one can imagine if the unit were tilted to the right, there is a close spacing between the nut 152 and the corner of the threaded bushing 140. This is highly reliable as long as that space is filled with a high dielectric fluid. However, if air were to be formed in that space, the possibility of arcing exists. Thus, as it turned out, leaving an air gap 158 is highly undesirable.

Although it is commonly known in the prior art to backfill large capacitors, power line transformers and the like with dielectric fluids, there is a need for active implantable medical devices which are filled with dielectric fluids. Backfilling of the housing of an AIMD with a dielectric fluid instead of a gas such as nitrogen would offer a number of important advantages including higher dielectric strength which means that the spacing between circuits can be reduced thereby permitting downsizing of the entire AIMD. In addition, backfilling with a dielectric fluid would have the advantage of providing an AIMD which is filled completely with relatively large molecules as compared with air, water, body fluids, or helium. Another advantage resides in the fact that in order to reduce weight and size, prior art AIMDs have very thin housing walls. This puts severe limits on recreational activities such as scuba diving or even playing baseball. Any pressure or sharp impact on the prior art AIMDs have been shown to deflect their housings and damage internal components. Another advantage of backfilling an AIMD with a dielectric fluid is a general increase in both reliability and circuit insulation resistance. Insulation resistance becomes very important along circuit traces and paths where a battery must last from five to fifteen years. Even a very small leakage current over a long period of time can significantly reduce the overall lifetime of an AIMD battery. Backfilling with a dielectric fluid also offers a very high degree of protection to internally installed components that may have been damaged during installation. For example, a surface mounted capacitor with a small micro-crack may, over long periods of time, form a dendrite and thereby a low insulation resistance or even a short circuit. However, the vacuum backfilling with a dielectric liquid tends to prevent the formation of any such long-term failure mechanisms even if a component defect such as a crack is present. Moreover, there is a continuing need to accommodate changes of pressure or temperature of the dielectric fluid in order to maintain housing or enclosure integrity. The present invention fulfills these needs, and provides other related advantages.

### SUMMARY OF THE INVENTION

The present invention resides in using dielectric fluids in order to increase the volts-per-mil breakdown strength of circuit elements and adjacent wires and circuit traces housed within an active implantable medical device (AIMD).

In accordance with the present invention, an AIMD generally comprises a housing having electronic components disposed therein. Dielectric fluid substantially fills the housing. The dielectric fluid has a dielectric breakdown strength (DBS) which exceeds that of commonly used backfilled gases, including air, nitrogen or helium. Typically, the dielectric fluid has a dielectric breakdown strength at least double that of air, nitrogen or helium under similar operating conditions, that is, similar AIMD and internal component arrangements, temperature, and pressure. Preferably, the dielectric fluid has a dielectric breakdown strength threshold of at least 100 volts per mil. Such dielectric fluid may comprise sulfur hexaflouride, mineral oil, or silicone oil.

Means are provided for accommodating change of pressure or temperature of the dielectric fluid to maintain housing integrity. Such accommodating means can comprise an expandable bellows attached to an outlet of the housing and in fluid communication with the dielectric fluid. Alternatively, at least one of the walls of the housing or a cap of the AIMD is resiliently flexible. A closed-cell foam material or resilient sphere may be disposed within the housing to accommodate for changes in temperature and/or pressure of the dielectric fluid. Alternatively, a recessed inlet/outlet has a resiliently flexible sealing member seated therein. In yet another alternative, an expandable baffle or bellows may be attached to an end cap so as to be in fluid communication with the dielectric fluid.

The AIMD may comprise a cardiac pacemaker, an implantable defibrillator, a congestive heart failure device, a hearing implant, a cochlear implant, a neurostimulator, a drug pump, a ventricular assist device, an insulin pump, a spinal cord stimulator, an implantable sensing system, a deep brain stimulator, an artificial heart, an incontinence device, a vagus nerve stimulator, a bone growth stimulator, a gastric pacemaker, or a Bion. The AIMD may comprise a bandstop filter assembly, including a hermetic seal assembly forming a housing and a capacitor and an inductor in parallel with one another. The AIMD may also comprise a feedthrough capacitor assembly, including a housing having a feedthrough capacitor therein, and a terminal pin extending through the housing and the capacitor. A bellows may be attached to an inlet/outlet of the housing and in fluid communication with the dielectric material to accommodate for changes in dielectric fluid pressure and/or temperature.

In accordance with the invention, a method for backfilling the active implantable medical device with a dielectric fluid comprises the steps of providing an AIMD having a housing with a fluid inlet/outlet. Substantially all of the air and moisture is evacuated from the housing through the housing inlet/outlet. This may be done by placing the housing of the AIMD within a vacuum chamber for a period of time. The housing may also be heated.

The housing is filled with the dielectric fluid having dielectric breakdown strength greater than that of air, nitrogen or helium. Typically, the dielectric breakdown strength is at least double that of air, nitrogen or helium under similar operating conditions, and typically the dielectric breakdown strength of the dielectric fluid is at least 100 volts per mil. The filling step includes the step of backfilling a portion of the vacuum chamber with the dielectric fluid, and then placing the dielectric fluid under a positive pressure by introducing an inert gas, such as nitrogen, into the vacuum chamber.

The housing inlet/outlet is sealed in a manner preferably accommodating expansion or contraction of the dielectric fluid in response to changes of pressure or temperature of the dielectric fluid. This may include providing an expandable baffle or bellows having an inlet in fluid communication with the AIMD. Alternatively, a resiliently flexible cap, plate or housing may be provided which is configured to deflect in response to changes in dielectric fluid pressure or temperature. A recessed inlet/outlet having a flexible sealing member associated therewith may also accommodate the changes in dielectric fluid pressure or temperature. The sealing member may comprise a sphere seated within the recess of an end cap. Alternatively, or in addition, the end cap may have a baffle or bellows associated therewith.

Additional means for accommodating changes of pressure or temperature of the dielectric fluid to maintain housing integrity include inserting a resiliently flexible member in the housing which contracts or expands in size in response to changes of pressure or temperature of the dielectric fluid. Such a resiliently flexible member may comprise a foam material or resilient sphere.

Other features and advantages of the present invention will become apparent from the following more detailed description, taken in conjunction with the accompanying drawings, which illustrate, by way of example, the principles of the invention.

### BRIEF DESCRIPTION OF THE DRAWINGS

The accompanying drawings illustrate the invention. In such drawings:
FIG. 1 is a wire formed diagram of a generic human body showing placement of a number of implanted medical devices;
FIG. 2A-2C are perspective views of two prior art implantable cardioverter defibrillators, and a smaller implantable cardiac pacemaker;
FIG. 3 is a perspective assembly illustration of a prior art layer-wound feedthrough capacitor;
FIG. 4 is an enlarged sectional view showing the layer-wound feedthrough capacitor of FIG. 3 installed in a metal housing;
FIG. 5 is a cutaway perspective view of a typical active implantable medical device such as those illustrated in FIGS. 2A-2C;
FIG. 6 is a cutaway perspective view similar to FIG. 5, wherein the interior is filled with a dielectric fluid in accordance with the present invention;
FIG. 7 is a sectional illustration of a prior art device known as a Bion;
FIG. 8 is an enlarged section of an end cap assembly which modifies the end cap shown in FIG. 7;
FIG. 9 is a top and side perspective view of the assemblies shown in FIG. 8 in a final assembled state;
FIG. 10 is a view similar to FIG. 8, showing an alternative bellows configuration;
FIG. 11 is a perspective view similar to FIG. 9, showing the assembly of FIG. 10;
FIG. 12 is an exploded perspective view of an assembly which includes an RFID chip placed within a hermetic housing suitable for human implant applications;
FIG. 13 shows an adaptation of the novel end cap to a bellows assembly;
FIG. 14 is a sectional view illustrating another methodology for encapsulating an AIMD end cap such as an RFID chip or a Bion;
FIG. 15 is an exploded perspective view of the structure of FIG. 14;
FIG. 16 is a top plan view of the assembly shown in FIG. 15;
FIG.17 is an enlarged fragmented view of the section taken along the line 17 in FIG. 16; and
FIG. 18 is an enlarged fragmented view taken along the line 18 in FIG. 16.
FIG. 19 is an enlarged, fragmented sectional view taken generally along the lines 19-19 of FIG. 4;
FIG. 20 is a perspective view of the bellows shown in FIG. 19;
FIG. 21 is a fragmented sectional view similar to that illustrated in FIG. 19 showing an alternative method for mounting the bellows;
FIG. 22 is an exploded perspective view of a portion of the assembly shown in FIG. 21;
FIG. 23 is a sectional view illustrating a hermetically sealed assembly for a bandstop filter;
FIG. 24 is an electrical schematic diagram of the bandstop filter of FIG. 23;
FIG. 25 is a view similar to FIG. 23, except that a miniature bellows assembly has been added;
FIG. 26 is a view similar to FIGS. 23 and 25 showing an alternative technique used for absorbing mechanical stresses from expansion and contraction of the enclosed dielectric fluid; and
FIG. 27 is a schematic flow chart illustrating a preferred method for backfilling an AIMD with dielectric fluid in accordance with the present invention.

### DETAILED DESCRIPTION OF THE PREFERRED EMBODIMENTS

The present invention, as shown in the accompanying drawings for purposes of illustration, resides in active implantable medical devices (AIMDs) which have been filled with a dielectric fluid, methods for backfilling the same, and providing protection means for expansion or contraction of said fluid.

AIMDs are typically backfilled with air or nitrogen which have relatively low dielectric breakdown strength thresholds (typically between 60-100 volts per mil). To prevent arcing and short circuiting or even damage to the internal electronic components of the AIMD, these components and circuits must be adequately spaced from one another. Of course, this presents several drawbacks, including an increased overall size of the AIMD.

Typical dielectric fluids, such as white mineral oil or silicone oil, have breakdown strengths that are well in excess of 1000 volts per mil. This means that the distance between circuit traces, electrical connections, flex cable wiring and circuit board layouts can all be significantly downsized within an AIMD if the AIMD housing is backfilled with such dielectric fluids, in accordance with the present invention.

One concern relating to the use of a dielectric fluid rather than a gas is the relatively higher weight or a specific gravity of fluids compared with air or backfilled gases such as nitrogen. However, this is offset by the dramatic reduction in size and volume of the AIMD due to the closer spacing of high voltage circuits. When one takes this into consideration, there is relatively little empty space left inside the AIMD. Accordingly, the weight of the injected fluid is relatively small.

Moreover, there are some dielectric gases that can be advantageously used in the present application, such as sulfur hexaflouride (chemical symbol SF₆). This particular gas is used in high voltage switches, including weapon switches. It is also used to suppress arcs in high voltage relays. Backfilling with this type of a gas does not increase the dielectric breakdown strength nearly as much as a liquid, but it is still about three times better than that of air or nitrogen. Accordingly, fluids as used herein include dielectric gases at room temperature with higher breakdown strength in comparison with air, nitrogen or helium.

Another important advantage of the present invention is that liquids with good dielectric properties tend to be very large molecules as compared to air, helium or nitrogen. This means that the hermetic seals used in implantable medical devices need not have the same stringent leak rate requirements as are necessary in a gas backfill environment. The large liquid molecules will not readily pass through even a glass hermetic seal. Thus, it may not be necessary to use expensive gold brazed ceramic seals and the like, which are commonly used in the prior art.

In addition to leak rate, there is also the consideration of time of implant. Normally cardiac pacemakers are only implanted for five to seven years. However, cochlear implants, for example, may be implanted for thirty or forty years, or longer. In prior art gas filled AIMDs, there often exists a differential pressure between the inside of the AIMD and the outside fluids. This leads to slow moisture intrusion over time. However, when the AIMD is backfilled with a liquid that consists of large molecules, this is no longer a serious concern. With a liquid filled system, there is no differential pressure.

In the prior art, with AIMDs such as cardiac pacemakers and implantable defibrillators, the hermetic seal is typically constructed of a sputtered and then gold brazed alumina ceramic to provide a seal that achieves a very low helium leak rate. Such AIMDs do not use much less expensive glass-to-metal seals that comprise either compression or fused glass seals. However, with the AIMD backfilled with a dielectric fluid in accordance with the present invention, one does not need as low a leak rate as is presently achieved by the very costly gold brazed alumina seals. Accordingly, it is a feature of the present invention that the hermetic seals may comprise a fused glass, a compression glass, or even a polycrystalline or polymer such as an epoxy.

In the foregoing and following descriptions, functionally equivalent components among the various illustrated embodiments will be designated by the same reference number.

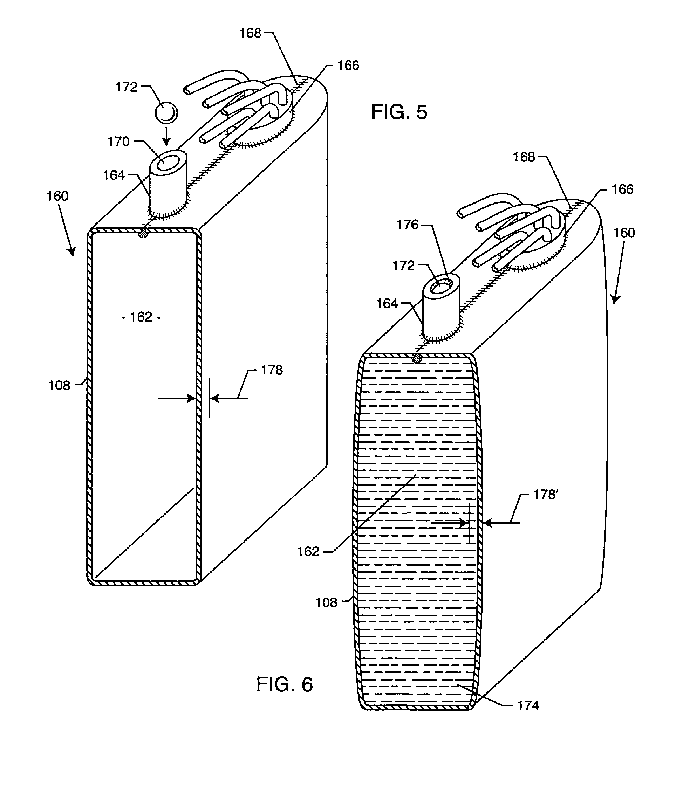

FIG. 5 is a cutaway view of a typical active implantable medical device (AIMD) 160 such as those previously illustrated in FIGS. 2A-2C for ICDs and cardiac pacemakers. One can see that it has a thin-wall titanium housing 108 as shown. In this case, the contents, which would include a battery, circuit board and other components, have been removed so that one can easily see the interior of the hollow cavity 162. There is a fill tube 164 which allows for impregnation of the AIMD 160. Typically this would be the last step in the process wherein the hermetic seal 160 and associated laser weld 168 have already been formed. In other words, the entire AIMD is hermetically sealed except through the hole 170 which passes through the center of the fill tube 164. The ball 172 has not been seated yet. In this way, the entire assembly, as shown in FIG. 5, can be immersed into an impregnation chamber.

In fact, in the prior art, it is typical that the entire assembly be filled with a nitrogen gas. This is in order to preclude moisture. However, there are a number of disadvantages to back filling the AIMD with nitrogen gas. For example, gases and moisture tend to be relatively small molecules when compared with a dielectric fluid, such as silicone oil. This means that the hermetic seal 166 must have a leak rate of at least 1 x 10⁻⁹ cc² per second. Another disadvantage of nitrogen is that the dielectric breakdown strength is very low compared to dielectric fluids. For example, a typical dielectric fluid such as silicone oil or mineral oil has a breakdown strength over 1000 volts per mil. In close gaps, air or nitrogen can break down at values at 100 or even 60 volts per mil.

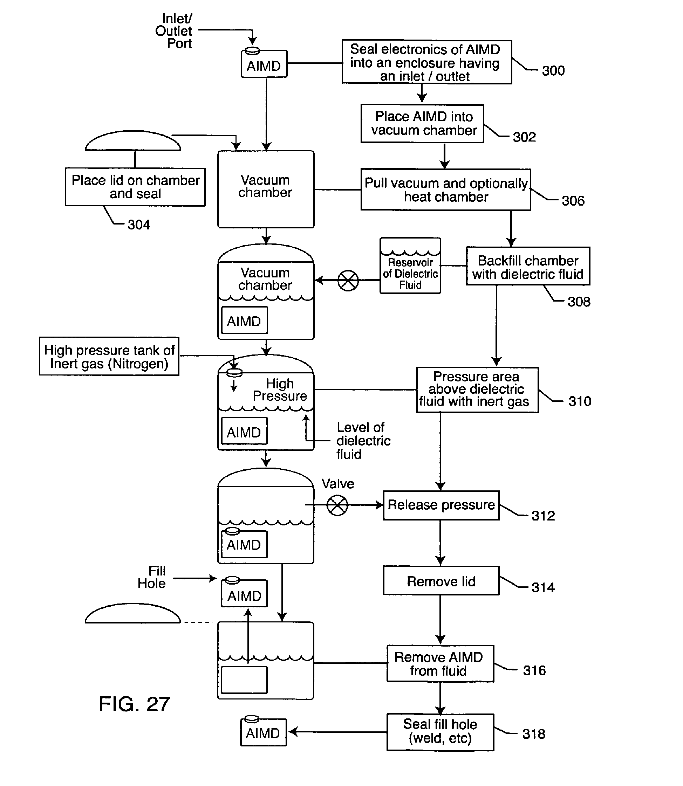

Referring now to FIG. 6, one can see the cutaway view of the AIMD 160 of FIG. 5 filled with a dielectric fluid 174. In this case, the ball 172 has been driven in the hole 170 after the impregnation process is complete and a laser weld 176 is formed to complete the hermetic sealing. In this way, the dielectric fluid 174 completely fills the interior spaces of the AIMD 160. In this process, shown schematically if FIG. 27, the AIMDs are placed first into a high temperature vacuum chamber and thoroughly evacuated, such as by exposing them to a vacuum for a time period. Then the dielectric fluid (which can also be certain types of dielectric gases) is flooded over the units which are typically held in some sort of a tray or a rack. This evacuation process can take many hours or even days. Then the dielectric fluid 174 is flooded into the chamber which completely covers the units. A positive pressure of dry hydrogen or other moisture free inert gas is typically placed on top of the reservoir of dielectric fluid to ensure that it reaches all of the interior spaces and cavities within the AIMD. At this point, the AIMD is removed from the dielectric fluid with the unit sitting upright, and then the sealing ball 172 is driven in such that no fluid 174 can escape during the final laser weld 176 hermetic sealing operation.

In this particular application, the thin-wall titanium can or housing 108 becomes a major advantage. This is because it is inherently flexible. That is, if the device is raised or lowered at temperature, the wall 108 of the housing is free to resiliently flex as shown at 178 in FIGS. 5 and 6. The wall as shown in FIG. 5 would be the neutral position for body temperature of 37 degrees centigrade. However, if the unit was heated up substantially (for example, in the trunk of a car on the way to the hospital), then the dielectric fluid 174 would tend to expand slightly. As shown in FIG. 6, this would not create any stress on the hermetic seal components 166 and 168 because the housing wall 108 would simply flex out to position 178'. There are a number of important advantages to backfilling an AIMD with a dielectric liquid. A first advantage is that circuit traces and circuit components can be placed much closer together. This is because the dielectric breakdown strength of such fluids is typically much greater when compared to air, nitrogen, helium or the like. None of these prior art backfill gases have very high breakdown strengths. This is particularly problematic in an implantable defibrillator application which must operate at very high voltages. Typical standoff distances for the high voltages inside the AIMD are about 25/1000 of an inch (25 mils). By backfilling with dielectric fluids, one can significantly reduce the size of circuit boards, circuit traces and the like. This downsizing would allow the ICD to be manufactured in a significantly smaller package. Size is very important for patient comfort since these devices are typically implanted in either the right or the left pectoral region on the patient's chest.

A disadvantage of using a dielectric liquid is that it would be heavier and add more weight to the finished device as compared with backfilling with a dielectric gas. This is offset in an ICD application, for example, by the fact that the device can be manufactured substantially smaller. By efficient internal component layout, the goal is to have relatively small internal air spaces that will be backfilled with the dielectric liquid. By using an efficient design of this manner, then the weight of the liquid becomes relatively small.

Referring back to FIG. 6, other advantages of a liquid impregnated AIMD include higher resistance to external mechanical shocks. It is a concern of AIMD manufacturers, doctors and patients that the pacemaker or ICD not be crushed or damaged. For example, during sports activities, such as baseball, one would not want a baseball to impact the relatively thin wall 108 of the AIMD. However, if the AIMD is completely backfilled with a liquid, it becomes much more resistant to external impacts. Another advantage lies in activities such as scuba diving. When the AIMD is backfilled with a gas, it is very sensitive to pressure differentials. However, when a pacemaker is backfilled with a liquid, then the current limitations on pacemaker patients as to how deep they can go when scuba diving would be removed or significantly modified. There are a number of other advantages that will be obvious to those skilled in the art.

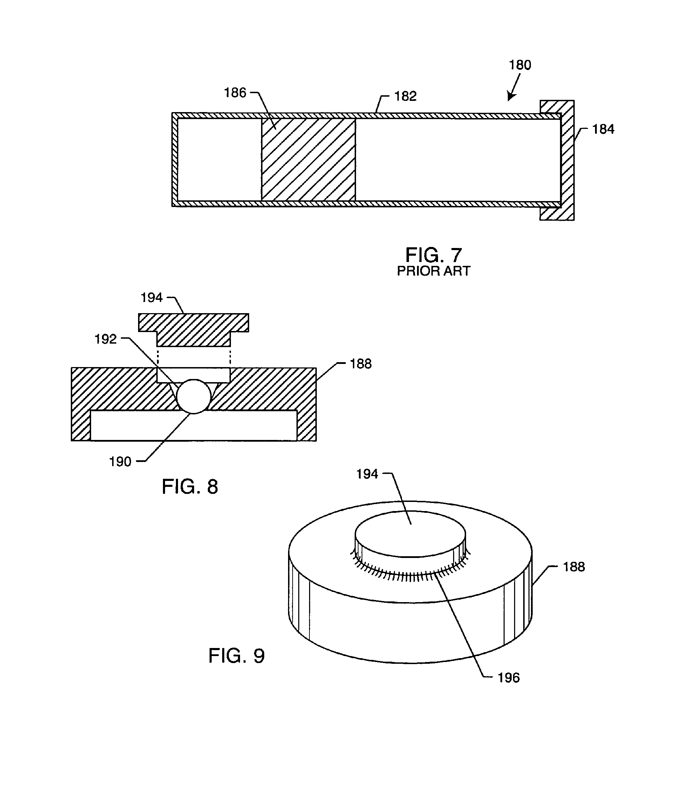

FIG. 7 illustrates a prior art device 180 which is known as a Bion. It has a ceramic tube 182 which typically has an end cap electrode 184 and then an electrode band 186. The Bions can be externally powered by a pulsing magnetic field or internally powered by their own battery. Bions are known in the art for neurostimulation. Typically Bions are backfilled with gas in a very similar manner as previously described for pacemakers, ICDs and other types of neurostimulators. It would be an advantage to backfill the Bion 180 of FIG. 7 with a dielectric fluid. However, this must be done in a way such that the dielectric fluid does not expand and fracture the delicate hermetic seals of the Bion 180 during temperature or pressure cycling.

FIG. 8 is a novel end cap assembly 188 which modifies the end cap of 184 from FIG. 7 such that it has a flexible sealing member, in the form of a sealing ball 190 disposed in a recessed inlet/outlet 192 of the cap 188 and a permanent end cap 194. This allows for a convenient method of backfilling the Bion of FIG. 7 with a dielectric fluid. During impregnation operations, the ball 190 and the end cap 194 would be left out. After the impregnation process and the Bions 180 are completely filled with fluid, the ball 190 would be driven in place to form a temporary seal. Then the end cap 194 would be seated and a laser weld 196 would be formed as shown in FIG. 9 there is heat associated with a laser weld or braze 196. The presence of the seated ball 190 enables the dielectric fluid to expand, while not interfering with the proper formation of the hermetic laser weld joint 190. Moreover, the ball or sphere 190, being resiliently flexible so as to contract or expand, accommodates for changes in temperature or pressure of the dielectric fluid within the Bion 180.

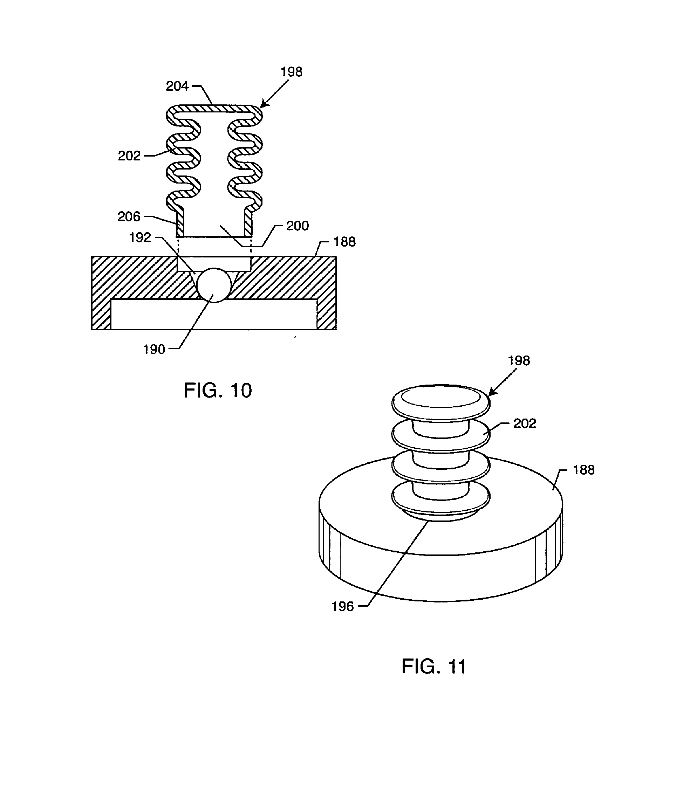

FIG. 10 is very similar to FIG. 8 except the sealing cap 194 has been replaced by a miniature expansion bellows assembly 198. The bellows 198 allows for expansion and contraction of the dielectric fluid without placing undue pressure on the hermetic seal joints. The bellows includes an open end 200 which is in fluid communication with the dielectric fluid. The bellows 198 is capable of being expanded and contracted. The bellows 198 may be comprised of a resiliently flexible metal. The bellows 198 includes a side wall 202 which is convoluted so as to be accordion-folded in configuration. The bellows 198 includes a closed end 204, as illustrated generally opposite the open end 200. As dielectric fluid increases in temperature or pressure, it enters into the bellows 198 and can push outwardly such that the convoluted or accordion folds 202 enlarge and become straightened. Preferably, the accordion-type folds or walls 202 will return to their natural folded state once the temperature or pressure decreases. A bottom flange 206 of the bellows 198 is inserted into the recessed inlet/outlet 192 so as to be fastened to the cap 188. Such fastening can be done by means of the weld 196 illustrated in FIG. 11, or can be by means of adhesive, threaded engagement, etc.

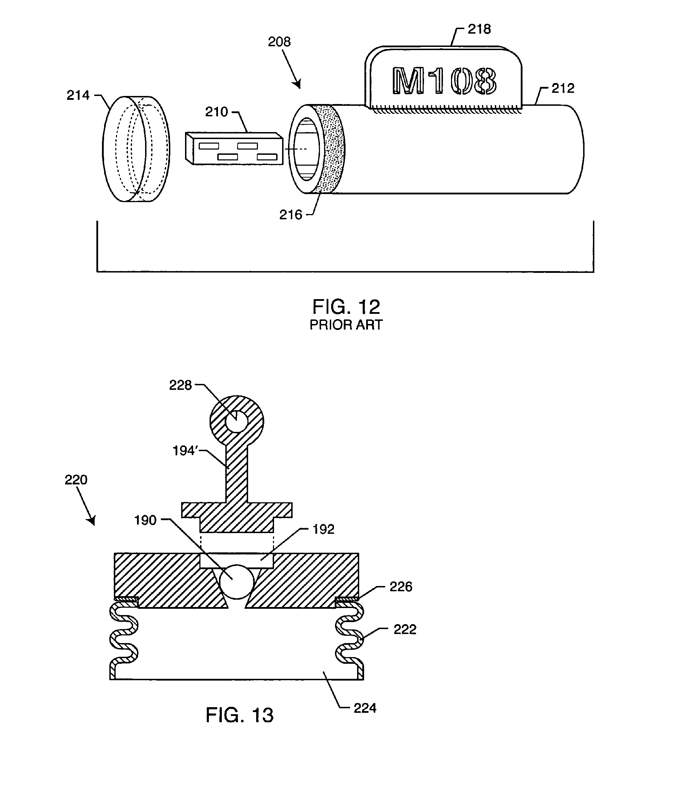

FIG. 12 shows an assembly 208 having an RFID chip 210 which has been placed into a hermetic housing 212 suitable for human implant. This is more completely described in U.S. Patent Application Serial No. 60/594,230, the contents of which are incorporated herein by reference. One can see that there is an end cap 214 which is designed to be gold brazed or laser welded to the metallization 216 of the ceramic tube 212 that forms the hermetic seal. Also shown is an optional X-ray identification tag 218.

As previously described herein, it would be desirable to have this entire assembly 208 backfilled with a dielectric fluid. The use of a dielectric fluid is particularly advantageous in extremely small AIMDs. This is because the cross sectional area of the hermetic seal is relatively large compared to its interior space. This causes problems in that over time body fluid may penetrate. In other words, smaller AIMDs like this have to have more robust hermetic seals. For example, for a relatively large unit, like a pacemaker, a helium leak rate during testing of 1 x 10⁻⁹ cc² per cm is acceptable. However, for an extremely small AIMD, leak rates of 1 x 10⁻¹² or even 1 x 10⁻¹⁴ are required. In accordance with the present invention, if the unit were to be 100% filled with a dielectric fluid, such as silicone oil, then these leak rates would not need to be nearly as low in value. This is because the silicone is a relatively large molecule. Silicone will not readily pass over time through a hermetic seal nearly as well as a gas, such as helium. Accordingly, it would be a major advantage in small AIMDs to be able to backfill them 100% with a dielectric fluid (as opposed to prior art gases, such as dry nitrogen).

FIG. 13 is a cross-sectional view of an end cap 220 which incorporates the teachings of the end caps of both FIGS. 8 and 10, described above. That is, the end cap 220 includes a sealing member, such as in the form of a ball or sphere 190 disposed within a recessed inlet/outlet 192 such that a cap 194' can be inserted thereover and at least partially into the recessed area 192. A bellows portion 222, having an open end 224 and a generally opposite end affixed to the end cap 220 such as by braze, adhesive, etc. 226. As such, the cap 220 acts as the closed end of the bellows assembly, otherwise the bellows is similar in that it has accordion-type folds which are capable of expanding and contracting. This would accommodate the change of temperature and/or pressure of the dielectric fluid within the AIMD 208. The modified end cap 194' may include a through-hole 228 which a physician could use to attach the device 208 to some body part or even to an implanted lead wire or the like for identification purposes, etc.

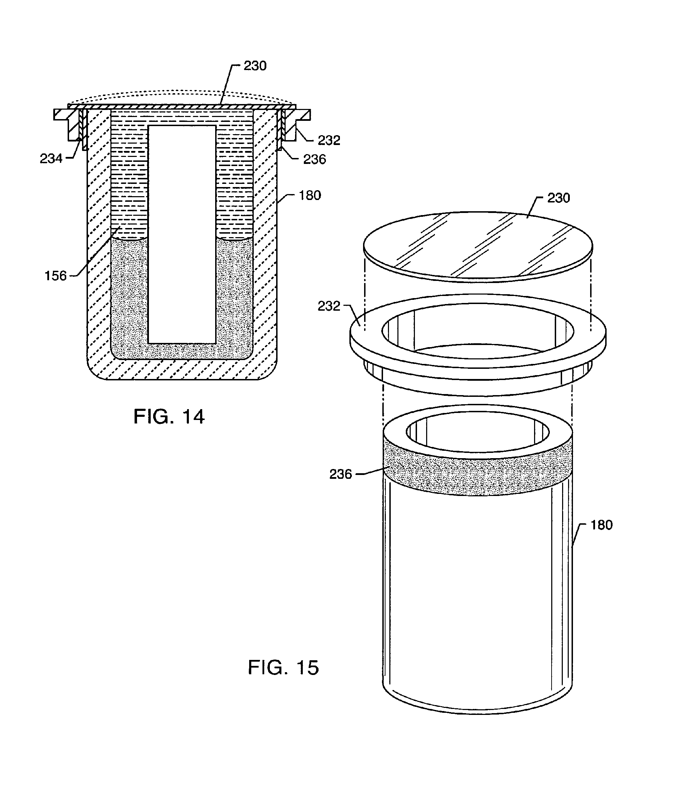

FIG. 14 illustrates another methodology of encapsulating an AIMD, such as a Bion. This is very similar to the Bion 180 as described in FIG. 7 except that a very thin end plate 230 has been laser welded into place. This extremely thin plate is designed to resiliently expand and contract as the dielectric fluid 156 expands and contracts. This is shown in exploded assembly view FIG. 15. There is a metallic ferrule 232 that is typically of titanium that has previously been gold brazed 234 to metallization 236 on the ceramic enclosure 180.

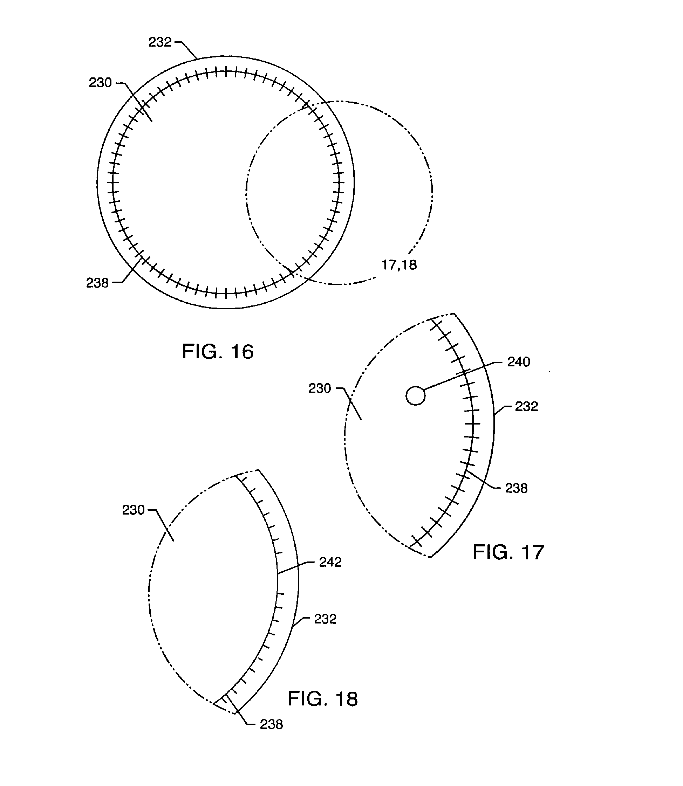

FIG. 16 shows a top view with a laser weld 238 that's been formed between the ferrule 232 and the end cap 230. FIG. 17 shows a novel small hole 240 which allows for impregnation. After the devices are removed from the impregnation chamber, then it is a relatively simple matter to use a laser welder to plug the small hole 240 before liquid could escape. FIG. 18 shows an alternative method of achieving the same purpose without the hole as shown in FIG. 17. In this case, a gap 242 has been left in the laser weld 238. This forms an area of the end plate 230 which has not been hermetically sealed to the ferrule 232. In a vacuum, this allows for the interior air to be evacuated and also for the dielectric fluid to penetrate during the impregnation process. After the impregnation process is completed, it is then a relatively simple matter to complete the rest of the laser weld and close the gap 242.

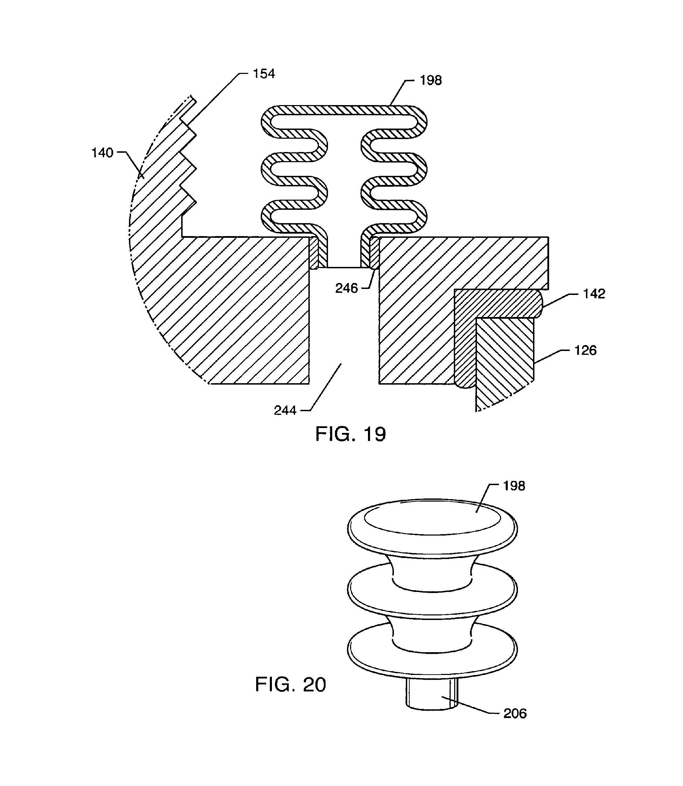

With reference now to FIGS. 19-22, the AIMD can comprise relatively small devices, such as a feedthrough capacitor, similar to that illustrated in FIG. 4. Prior attempts to fill such small capacitors with dielectric fluid resulted in failure of the devices due to fluctuations in temperature and pressure of the dielectric fluid, which caused the integrity of seals and joints and the like to fail. In accordance with the present invention, means can be provided to accommodate such fluctuations of temperature and/or pressure of the dielectric fluid. A miniature bellows assembly 198, as previously illustrated and described, can have a lower open end 206 thereof inserted into a passageway 244 which is in fluid communication with the dielectric fluid. In this manner, the thin-wall bellows assembly 198 is in fluid communication with the dielectric fluid, which allows for expansion and contraction of the fluid due to temperature and pressure variations without putting undue forces on surrounding structures. The use of a bellows 198 also eliminates the problems that would be associated with an entrapped air bubble.

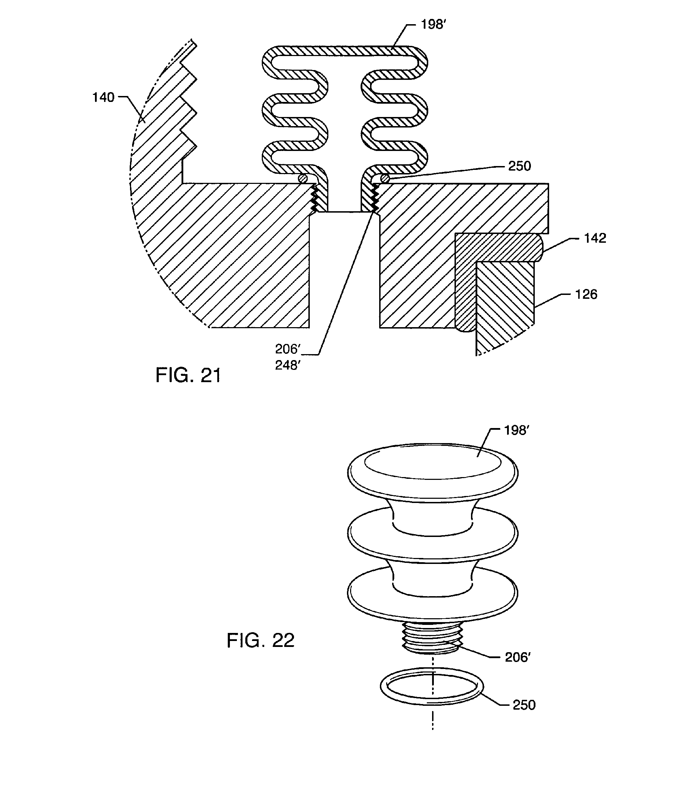

In FIGS. 19 and 20, the end 206 of the bellows 198 is generally smooth so as to be affixed to the capacitor assembly by means of a solder or braze 246. FIGS. 21 and 22 illustrate a bellows assembly 198' having a threaded end 206' which is threadedly received within an internally threaded portion 248 of the passageway. Preferably, the threaded bellows 198' is threaded against an O-ring 250 to ensure a fluid-tight seal. By using a threaded bellows 198', a number of efficiencies are achieved. During the impregnation process, it is very easy to leave the bellows 198' out. After the impregnation process is completed and with the bellows 198' also full of dielectric fluid, it is a relatively simple matter to screw it into place which completes the entire assembly without the need for additional heat processes.

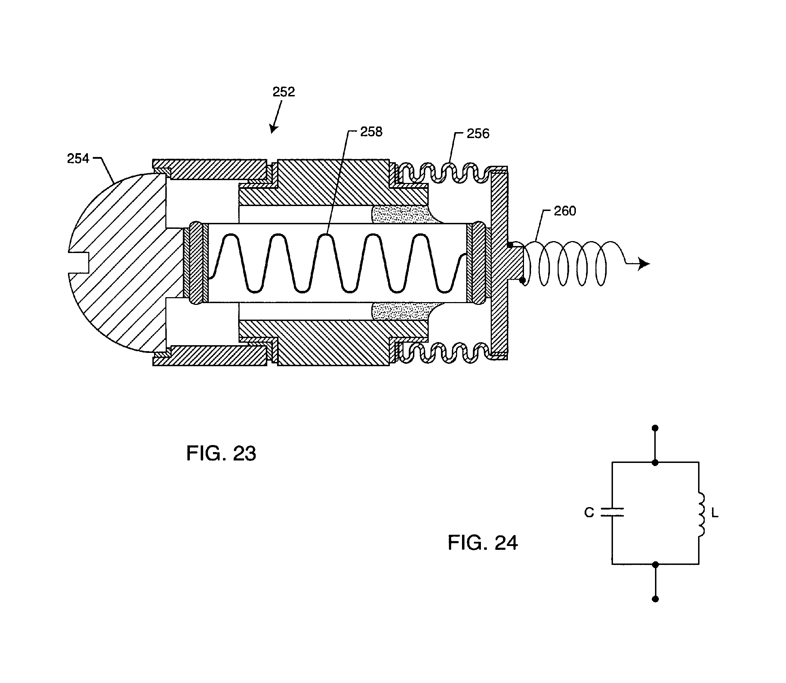

FIG. 23 illustrates a hermetically sealed assembly of a bandstop filter 252. Bandstop filters, when used in conjunction with an implanted lead wire, are very useful to prevent overheating of said lead wire during medical diagnostic procedure, such as MRI. This is more thoroughly described in U.S. Patent No. 7,363,090 and in pending U. S. Patent Publication Nos. 2007-0112398 A1, 2007-0288058 A1, 2008-0077241 A1, 2008-0049376 A1, 2008-0132987 A1, and 2008-0116997 A1, and U.S. Patent Application Serial No. 61/016,364, the contents of all of which are incorporated by reference herein.

FIG. 24 is a schematic diagram showing the parallel capacitor (C) and inductor (L) components of the bandstop filter 252 of FIG. 23. FIG. 23 is completely described in U.S. Patent Publication No. 2007-0112398. It has a distal electrode 254 which in this case is a passive electrode. This could also be an active screw-in helix electrode. There is a hermetic seal assembly formed between the insulator metallic end rings and various gold brazes and laser welds to perform hermetic seals. Shown is a novel bellows assembly 256 which when combined with flexible electrical connections 258 and 260 allow for expansion and contraction of the assembly. A small gap (not shown) would be left in one laser weld. This small gap would be left to allow for proper evacuation and impregnation of a dielectric fluid. This small gap would then be filled after the units were removed from the impregnation chamber by placing a small laser weld.

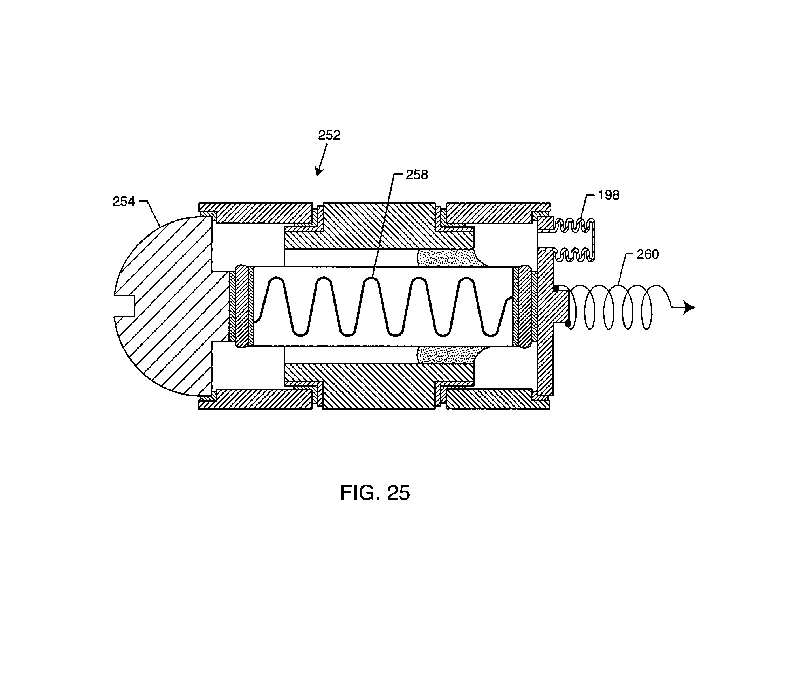

FIG. 25 is very similar to FIG. 23 except that a miniature bellows assembly 198' has been added and is suitable for laser welding into the end of the housing after the unit is impregnated.

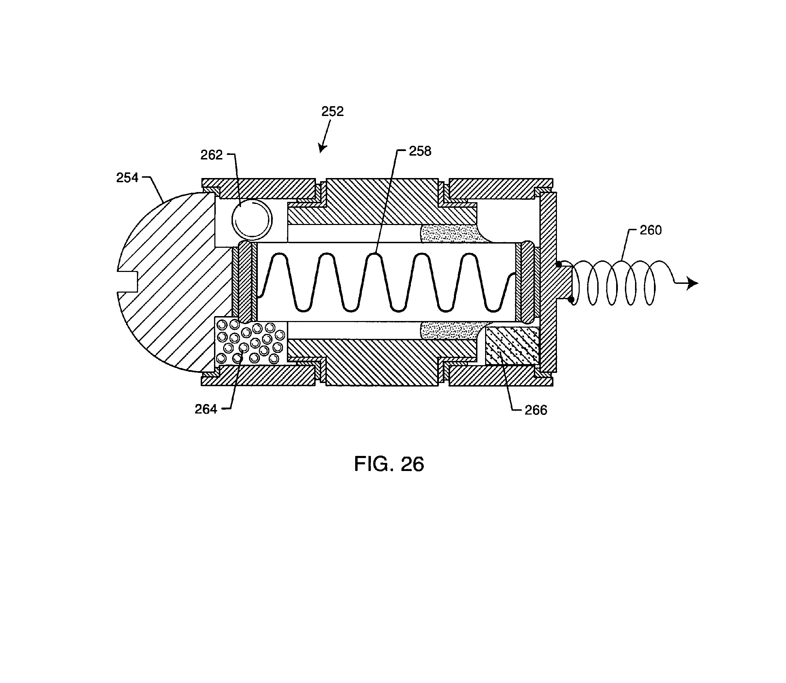

FIG. 26 is also very similar to FIG. 25 except that it has been completely encapsulated. In this case, there are a number of alternative techniques that are used to absorb and accommodate the mechanical stresses from the expansion and contraction of the enclosed dielectric fluid. This could be resiliently flexible members disposed within the housing which contract or expand in size in response to changes of pressure or temperature of the dielectric fluid. These may include thin hollow spheres or rubber spheres 262, small micro-spheres 224 or even a closed solid foam or neoprene structure 266. By having a small area that has such flexibility, as the fluid expands and contracts, it would not put undue pressure on the hermetic seal joints. It will be obvious to those skilled in the art that these embedded types of expansion and contraction devices 262-266 are also applicable to any other type of AIMD or any other drawings as previously described herein.

As previously mentioned, FIG. 27 is a schematic flow chart illustrating a preferred method for backfilling an AIMD with dielectric fluid in accordance with the present invention. The construction of an AIMD is shown at 300 wherein the electronics of the AIMD are enclosed within an overall hermetically sealed housing, and an inlet/outlet port is left to facilitate impregnation of the AIMD with the dielectric fluid. In the prior art, such as cardiac pacemakers, the AIMD housing would typically be of stainless steel. There are other types of AIMDs that even use ceramic housings. The AIMD is placed into a vacuum chamber as shown at 302, and normally has very thick walls and viewing ports, and a heavy lid which is designed to be seated onto an O-ring and then clamped firmly in place. The AIMD is placed into the vacuum chamber and then the sealing lid is placed so that the chamber can be evacuated as shown at 304. Vacuum pumps pull a vacuum within the vacuum chamber and heat is optionally applied to the outside of the chamber (through heating coils) at the same time as shown at 306. This step can be just a few hours or many days. The vacuum pulls out all of the entrapped air and moisture from all internal cavities inside the AIMD housing through the inlet/outlet port.

Once the AIMD has been held at sufficiently low pressure and heat for a sufficient period of time, then in step 308 the chamber is flooded to a height so that the entire AIMD is covered in the dielectric fluid of choice. Soon thereafter, referring to step 310, a valve is opened such that high pressure inert gas is flooded into the chamber above the level of the dielectric fluid. Typically, this would be of dry nitrogen. This positive pressure, for example, could be 35, 60 or even 90 PSI. This creates a huge pressure differential inside of the AIMD. At this moment, the inside cavities of the AIMD are at a hard vacuum. With the top of the dielectric fluid pressurized, this tends to drive the dielectric fluid into every pore and space inside of the AIMD and more importantly between every area where there is a circuit trace. This is facilitated by optionally keeping the chamber heated so that the viscosity of the dielectric fluid is low. Preferably, the pressure would be kept on top of the dielectric fluid layer for several hours as the heating coils that surround the vacuum chamber are turned off. This allows the dielectric fluid to completely cool before the pressure is released (312). This is important so that when the AIMD is removed from the chamber that the fluid will not be subjected to any differential pressure which might cause some of it to seep out.

After the chamber has cooled, a valve is opened to release the pressure on top of the dielectric fluid (312). The lid is then removed (314). The AIMD is then removed from the dielectric fluid (316). During this process, which is normally done by removing an entire rack, the AIMD must be kept in a vertical position with its fill hole (inlet/outlet) pointing upward. It would be highly undesirable for it, for example, to tip over and have dielectric fluid leak out. The fill hole is then hermetically sealed by laser welding or the like (318).

Although several embodiments have been described in detail for purposes of illustration, various modifications may be made without departing from the scope and spirit of the invention. Accordingly, the invention is not to be limited, except as by the appended claims.

## Claims

1. A method for backfilling an active implantable medical device (AIMD) with a dielectric fluid, comprising the steps of:
providing an AIMD having a housing with a fluid inlet/outlet;
evacuating substantially all air and moisture from the housing through the housing inlet/outlet;
filling the housing with a dielectric fluid having a dielectric breakdown strength greater than air, nitrogen or helium; and
sealing the housing inlet/outlet.

2. The method of claim 1, wherein the AIMD comprises a cardiac pacemaker, an implantable defibrillator, a congestive heart failure device, a hearing implant, a cochlear implant, a neurostimulator, a drug pump, a ventricular assist device, an insulin pump, a spinal cord stimulator, an implantable sensing system, a deep brain stimulator, an artificial heart, an incontinence device, a vagus nerve stimulator, a bone growth stimulator, a gastric pacemaker, or a Bion.

3. The method of claim 1, wherein the evacuating step comprises the step of placing the housing of the AIMD within a vacuum chamber for a period of time.

4. The method of claim 3, wherein the evacuating step includes the step of heating the housing.

5. The method of claim 3, wherein the filling step comprises the step of backfilling a portion of the vacuum chamber with the dielectric fluid.

6. The method of claim 5, including the step of placing the dielectric fluid under a positive pressure.

7. The method of claim 6, including the step of introducing an inert gas into the vacuum chamber.

8. The method of claim 7, wherein the inert gas comprises nitrogen.

9. The method of claim 1, wherein the dielectric fluid has dielectric breakdown strength at least double that of air, nitrogen or helium under similar operating conditions.

10. The method of claim 1, wherein the dielectric fluid has dielectric breakdown strength of at least 100 volts per mil.

11. The method of claim 1, wherein the filling step comprises the step of filling the housing with at least one of sulfur hexaflouride, mineral oil, or silicone oil.

12. The method of claim 1, including the step of accommodating changes of pressure or temperature of the dielectric fluid to maintain housing integrity.

13. The method of claim 12, wherein the sealing step includes the step of accommodating expansion or contraction of the dielectric fluid in response to changes of pressure or temperature of the dielectric fluid.

14. The method of claim 13, wherein the accommodating step includes the step of providing an expandable bellows having an inlet in fluid communication with the AIMD.

15. The method of claim 13, wherein the accommodating step includes the step of providing a resiliently flexible housing, plate or cap configured to deflect in response to changes in dielectric fluid pressure or temperature.

16. The method of claim 12, wherein the accommodating step includes the step of inserting a resiliently flexible member in the housing which contracts or expands in size in response to changes of pressure or temperature of the dielectric fluid.

17. The method of claim 16, wherein the member comprises a foam material or a resilient sphere.

18. The method of claim 12, wherein the accommodating step includes the step of providing a recessed inlet/outlet having an flexible sealing member associated therewith.

19. The method of claim 18, wherein the sealing member comprises a sphere seated within a recess of an end cap.

20. The method of claim 12, wherein the accommodating step includes the step of providing a bellows associated with an end cap.

21. An active implantable medical device (AIMD), comprising: a housing;
electronic components disposed within the housing;
dielectric fluid substantially filling the housing, the dielectric fluid having a dielectric breakdown strength exceeding that of air, nitrogen, or helium; and means for accommodating change of pressure or temperature of the dielectric fluid to maintain housing integrity.

22. The AIMD of claim 21, wherein the AIMD comprises a cardiac pacemaker, an implantable defibrillator, a congestive heart failure device, a hearing implant, a cochlear implant, a neurostimulator, a drug pump, a ventricular assist device, an insulin pump, a spinal cord stimulator, an implantable sensing system, a deep brain stimulator, an artificial heart, an incontinence device, a vagus nerve stimulator, a bone growth stimulator, a gastric pacemaker, or a Bion.

23. The AIMD of claim 21, wherein the dielectric fluid has a dielectric breakdown strength at least double that of air, nitrogen or helium under similar operating conditions.

24. The AIMD of claim 21, wherein the dielectric fluid has a dielectric breakdown strength of at least 100 volts per mil.

25. The AIMD of claim 21, wherein the dielectric fluid comprises at least one of sulfur hexaflouride, mineral oil, or silicone oil.

26. The AIMD of claim 21, wherein the accommodating means comprises an expandable bellows attached to an outlet of the housing and in fluid communication with the dielectric fluid.

27. The AIMD of claim 21, wherein the accommodating means comprises at least one of the walls of the housing being resiliently flexible.

28. The AIMD of claim 21, wherein the accommodating means comprises a resiliently flexible cap of the AIMD.

29. The AIMD of claim 21, wherein the accommodating means comprises a closed-cell foam material or a resilient sphere disposed within the housing.

30. The AIMD of claim 21, wherein the accommodating means comprises a recessed inlet/outlet having an resiliently flexible sealing member seated therein.

31. The AIMD of claim 30, wherein the accommodating means comprises an expandable bellows attached to an end cap and in fluid communication with the dielectric fluid.
